(19) Europäisches Patentamt
European Patent Office
Office européen des brevets

(11) Veröffentlichungsnummer: **0 218 980 B1**

(12) # EUROPÄISCHE PATENTSCHRIFT

(45) Veröffentlichungstag der Patentschrift: **29.07.92**

(51) Int. Cl.$^5$: **C07D 209/50**

(21) Anmeldenummer: **86113418.7**

(22) Anmeldetag: **30.09.86**

Die Akte enthält technische Angaben, die nach dem Eingang der Anmeldung eingereicht wurden und die nicht in dieser Patentschrift enthalten sind.

(54) **Verfahren zur Herstellung von Tetrachlor-3-imino-isoindolin-1-on.**

(30) Priorität: **03.10.85 DE 3535276**

(43) Veröffentlichungstag der Anmeldung:
**22.04.87 Patentblatt 87/17**

(45) Bekanntmachung des Hinweises auf die Patenterteilung:
**29.07.92 Patentblatt 92/31**

(84) Benannte Vertragsstaaten:
**CH DE FR GB LI**

(56) Entgegenhaltungen:
**DE-A- 2 250 852**
**DE-A- 2 850 782**
**FR-A- 1 446 964**

**JOURNAL OF ORGANIC CHEMISTRY, Band 41, Nr. 23, Februar 1976, Seiten 3769,3770; J.H. HALL: "A simple method for converting nitriles to amides, hydrolysis with potassium hydroxide in tert-butyl alcohol"**

(73) Patentinhaber: **BASF Aktiengesellschaft**
**Carl-Bosch-Strasse 38**
**W-6700 Ludwigshafen(DE)**

(72) Erfinder: **Hahn, Erwin, Dr.**
**Am Buechsenackerhang 31**
**W-6900 Heidelberg(DE)**
Erfinder: **Kowarsch, Heinrich, Dr.**
**Kuernbacher Strasse 34**
**W-7519 Oberderdingen(DE)**

**EP 0 218 980 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von Tetrachlor-3-imino-isoindolin-1-on (I) oder dessen Alkalimetallsalz (Ia) durch Umsetzung von Tetrachlor-ortho-phthalodinitril mit einem Alkalimetallhydroxid in einem organischen Lösungsmittel und gewünschtenfalls Freisetzung von (I) aus (Ia) mittels verdünnter Säure.

Die DE-B-22 50 852 lehrt die Herstellung von Tetrachlor-3-imino-isoindolin-1-on (I) durch partielle Hydrolyse und Ringschluß des Tetrachlor-ortho-phthalodinitril (II) in Gegenwart von Alkalimetallen, wobei als Lösungsmittel vorteilhaft ein 1:1-Gemisch aus Wasser und organischem Lösungsmittel verwendet wird und als organische Lösungsmittel Alkohole wie Methanol, Ethanol und Isopropanol dienen. Tertiäre Alkohole werden nicht genannt. Die so erhaltenen Reinausbeuten liegen bei ca. 80 % bezogen auf (II). Deutlich niedrigere Ausbeuten werden erhalten, wenn man den Wassergehalt des Lösungsmittelgemisches reduziert (Vergleichsbeispiel 1 mit 67 % und Beispiel 7) und insbesondere wenn man die Umsetzung in wasserfreien Alkoholen durchführt. So ergibt die Umsetzung von (II) mit Natrium- bzw. Kaliumhydroxid in wasserfreiem Ethanol bzw. Methanol gemäß Beispiel 14 und 15 Ausbeuten von lediglich 43,5 bzw. 51 % an Natrium- bzw. Kaliumsalz von (I).

Ein hinsichtlich Ausbeute und Reinheit verbessertes Verfahren beschreibt die DE-B-28 50 782. Danach wird das Halogen-ortho-phthalodinitril gleichzeitig mit Ammoniak und Wasserstoffperoxid in einem Gemisch aus Wasser und einem organischen Lösungsmittel umgesetzt. Da bei dieser Reaktion in kurzer Zeit große Mengen Sauerstoff freigesetzt werden, ist das Verfahren technisch nicht unbedenklich. So wird in Org. Synthesis Coll. Vol. II. 586 (1943) über eine heftige Explosion bei der Hydrolyse von o-Tolunitril mit $H_2O_2$ berichtet.

In J. Org. Chem. 41, 3769 (1976) wird die Überführung einfacher Mononitrile, darunter Benzonitril in die entsprechenden Carbonamide durch Hydrolyse mit Natrium- oder Kaliumhydroxid in wasserfreiem Methanol oder tertiär-Butanol beschrieben. Die Ausbeute an Benzamid beträgt etwa 90 %. Mononitrile sind schon deshalb mit den hier einzusetzenden Dinitrilen nicht zu vergleichen, da kein Ringschluß erfolgt.

Der Erfindung lag nun die Aufgabe zugrunde, ein technisch einfaches und sicher durchzuführendes Verfahren bereitzustellen, das das Tetrachlor-3-imino-isoindolin-1-on (I) bzw. dessen Alkalimetallsalz (Ia) in hohen Ausbeuten bei guter Reinheit liefert.

Dementsprechend wurde ein Verfahren zur Herstellung von Tetrachlor-3-imino-isoindolin-1-on (I)

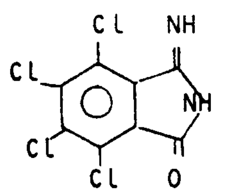

(I)

oder dessen Lithium-, Natrium- oder Kaliumsalz (Ia) durch Umsetzung von Tetrachlor-orthophthalodinitril (II)

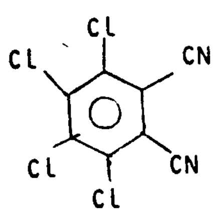

(II)

mit Lithium-, Natrium- oder Kaliumhydroxid in einem Alkohol als Lösungsmittel und gewünschtenfalls Freisetzung von (I) aus (Ia) mittels verdünnter Säure gefunden, das dadurch gekennzeichnet ist, daß man hierbei tert.-Butanol mit einem Wassergehalt von 0 bis 5 Gew.-% als Lösungsmittel verwendet.

Die erfindungsgemäße Umsetzung wird durch folgende Reaktionsgleichung beschrieben:

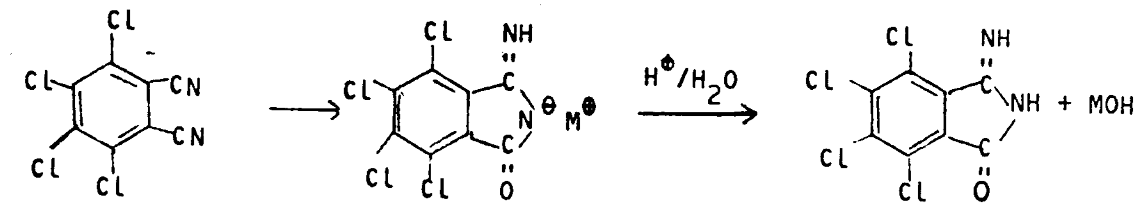


(II) (Ia) (I)

M = Li, Na, K

Das als Ausgangsmaterial verwendete Dinitril (II) kann leicht in bekannter Weise z.B. nach den in DE-C-16 43 744 und DE-C-19 32 421 beschriebenen Chlorierungsverfahren aus ortho-Phthalodinitril hergestellt werden, wonach (II) in Reinheiten zwischen 92 und 99 % anfällt.

Im Hinblick auf die Lehre der DE-B-22 50 852 ist es überraschend, daß die Hydrolyse von (II) in wasserfreiem bzw, weitgehend wasserfreiem tertiär-Butanol (d.h tertiar-Butanol mit 0-5 Gew. % Wasser) zu einer deutlichen Ausbeutesteigerung gegenüber der Umsetzung in einem wäßrig-organischen Medium von ca. 80% auf mehr als 90 % führt und daß die Hydrolyse in tertiär-Butanol wesentlich vorteilhafter verläuft als in anderen wasserfreien Alkoholen wie Ethanol oder Methanol.

Der Wassergehalt des im erfindüngsgemäßen Verfahren verwendeten tertiär-Butanol liegt vorteilhaft unter 5 Gew. %, vorzugsweise unter 1 %. Mit steigendem Wassergehalt gehen die Ausbeuten zunehmend zurück.

Die Menge an tertiär-Butanol ist nicht kritisch. Es wird zweckmäßigerweise soviel Lösungsmittel verwendet, daß das Reaktionsgemisch vor, während und nach der Umsetzung rührbar bleibt. Vorzugsweise kann man die 5- bis 15-fache, insbesondere die 6- bis 10-fache Gewichtsmenge tertiär-Butanol bezogen auf (II) verwenden.

Als Alkalimetallhydroxide kommen Lithium-, Natrium- oder Kaliumhydroxid oder Gemische dieser Hydroxide in Betracht. Die Menge an Alkalimetallhydroxid kann in weiten Grenzen variiert werden, zur Erzielung hoher Ausbeuten sollten jedoch nicht weniger als 1 mol pro Mol (II) verwendet werden. In der Regel werden 1 bis 5 mol, insbesondere 1,1 bis 2,5 mol, vorzugsweise 1,2 bis 2,0 mol Alkalimetallhydroxid je Mol Tetrachlor-ortho-phthalodinitril (II) verwendet. Vorteilhaft kann das Hydroxid dem Reaktionsgemisch in feingepulverter Form zugegeben werden.

Die Umsetzung kann zweckmäßigerweise bei Normaldruck in flüssigem tertiär-Butanol durchgeführt werden. Die Umsetzungstemperaturen werden daher durch Schmelz- und Siedepunkt des Lösungsmittels begrenzt. Vorteilhaft kann die Umsetzung bei Temperaturen von 26 bis 80, insbesondere bei 40 bis 70, vorzugsweise bei 45 bis 55 ° C durchgeführt werden. Bei Temperaturen oberhalb von 40 ° C ist die Reaktion in der Regel nach 1 bis 2 Stunden beendet.

Das nach der Umsetzung im Reaktionsgemisch vorliegende Alkalimetallsalz von (I) kann in an sich bekannter Weise z.B durch Filtration isoliert werden. Falls die Überführung in das Tetrachlor-3-imino-isoindolin-1-on gewünscht wird, kann man entweder das rohe Reaktionsgemisch ansäuern oder vorteilhaft das Salz zunächst abtrennen und dann mit einer Säure umsetzen. Die Freisetzung von (I) aus dem Salz erfolgt nach bekannten Methoden, z.B. durch Umsetzung mit verdünnten Mineralsäuren oder organischen wasserlöslichen Säuren wie Essigsäure.

Zweckmäßigerweise kann man das Verfahren so durchführen, daß man das Dinitril (II) in das weitgehend wasserfreie tertiär-Butanol gibt und dann unter Rühren das feinteilige Alkalimetallhydroxid zufügt. Nach der Umsetzung im oben angegebenen Temperaturbereich wird das Reaktionsgemisch vorteilhaft auf ca. 25 ° C abgekühlt und filtriert. Das feste Alkalimetallsalz kann dann z.B. durch Eintragen in verdünnte Säure in das freie Tetrachlor-3-imino-isoindolin-1-on überführt werden.

Durch Verwendung von weitgehend wasserfreiem tertiär-Butanol als Lösungsmittel ist es vorteilhaft möglich, das Filtrat mehrere Male, vorzugsweise 1 bis 5 mal, als Reaktionsmedium wiederzuverwenden, ohne daß eine Reinigung erforderlich ist. Die Rückführung des tertiär-Butanol ermöglicht eine besonders wirtschaftliche Verfahrensweise.

Nach dem erfindungsgemäßen Verfahren kann das Tetrachlor-3-imino-isoindolin-1-on (I) in Reinausbeuten von 95 bis 98 % bezogen auf das Ausgangsmaterial (II) erhalten werden. Die Bestimmung des Gehaltes an (I) erfolgt durch Titration mit Perchlorsäure. Die Reinheit des Produktes, die im allgemeinen bei ca. 95 % liegt, wird durch HPLC (Hochdruckflüssigkeitschromatographie, reversed phase; Rosil 5 $\mu$; externer Stan-

dard) ermittelt.

(I) dient als Zwischenprodukt zur Herstellung von Pigmenten mit hoher Lichtechtheit und guter Hitzebeständigkeit.

Beispiel 1

In einem Kolben wurden 400 g flüssiges tertiär-Butanol und 53 g (= 0,2 mol) Tetrachlor-ortho-phthalodinitril vorgelegt. Nach Zugabe von 17 g (= 0,3 mol) feingepulvertem KOH wurde die Reaktionsmischung 2 h bei 48 - 52 °C gerührt. Anschließend wurde auf 20 °C abgekühlt und der Feststoff auf einer Nutsche filtriert.

Das aufgefangene Filtrat (ca. 330 g) wurde durch frisches tertiär-Butanol ergänzt und als Reaktionsmedium wiederverwendet.

Das Nutschgut wurde in 400 ml Wasser suspendiert, das Gemisch durch Zugeben von verdünnter Essigsäure auf pH ≈ 6 gestellt, der Feststoff abgesaugt, mit Wasser gewaschen und im Vakuum getrocknet.

Ausbeute: 54 g Tetrachlor-3-imino-isoindolin-1-on (= 95 %)
HPLC-Reinheit = 97,5 %.

Beispiel 2

Das Verfahren von Beispiel 1 wurde wiederholt, jedoch wurde anstelle von Kaliumhydroxid feinteiliges Natriumhydroxid verwendet.

Ausbeute: 52 g Tetrachlor-3-imino-isoindolin-1-on (= 92 %)
HPLC-Reinheit = 95 %).

Beispiel 3

a) Es wurde wie in Beispiel 1 verfahren.
Das nach dem Abtrennen des Umsetzungsproduktes erhaltene tertiär-Butanol-Filtrat(= 330 g) wurde durch Zufügen von reinem tertiär-Butanol auf 400 g ergänzt und für eine weitere Umsetzung entsprechend Beispiel 1 verwendet.
Ausbeute: 56 g Tetrachlor-3-imino-isoindolin-1-on (= 98 %)
HPLC-Reinheit = 95 %.

b) Das nach dem Abtrennen des Umsetzungsproduktes unter a) erhaltene tertiär-Butanol-Filtrat wurde, auf 400 g ergänzt und für eine weitere Umsetzung entsprechend Beispiel 1 verwendet.
Ausbeute: 55 g Tetrachlor-3-imino-isoindolin-1-on (= 96,7 %)
HPLC-Reinheit = 93 %.

c) Das nach Abtrennung des Umsetzungsproduktes unter b) erhaltene tertiär-Butanol-Filtrat wurde auf 400 g ergänzt und für eine weitere Umsetzung entsprechend Beispiel 1 verwendet.
Ausbeute: 54 g Tetrachlor-3-imino-isoindolin-1-on (= 95 %)
HPLC-Reinheit = 92 %.

Vergleichsbeispiel 1 gemäß DE-B-28 50 782

5 Teile Natriumhydroxid werden in 12,5 Teilen Wasser aufgelöst und unter gründlichem Rühren mit 75 Teilen Ethanol versetzt. Zu der Lösung werden 20 Teile 3,4,5,6-Tetrachlorphthalodinitril gegeben, worauf man 5 Stunden bei 70 bis 78 °C rührt, dann das Reaktionsgemisch auf 15 °C abkühlt und durch Absaugen filtriert. Der erhaltene Kuchen wird mit Wasser gewaschen und dann zu 100 Teilen einer 5 %igen wäßrigen Essigsäure gegeben. Das Gemisch wird gerührt und durch Absaugen filtriert, worauf man den erhaltenen Kuchen gründlich mit Wasser wäscht und unter vermindertem Druck trocknet. Dabei erhält man 7,16 Teile eines Feststoffs, der als 3-Imino-4,5,6,7-tetrachlor-isoindolin-1-on identifiziert wird. Die HPLC-Reinheit liegt bei 94,0 %. Die Ausbeute beträgt 67 %.

Vergleichsbeispiel 2

Es wurde wie in Beispiel 1 verfahren, jedoch anstelle von tertiär-Butanol ein Lösungsmittelgemisch aus Wasser und tertiar-Butanol verwendet. Wie der folgenden Tabelle zu entnehmen ist, sinkt die Ausbeute mit zunehmendem Wassergehalt.

| Beispiel | tert.Butanol [g] | $H_2O$ [g] | Ausbeute [%] |
|---|---|---|---|
| 1 | 400 | 0 | 95 |
| 2a | 396 | 4 | 94 |
| 2b | 380 | 20 | 91 |
| 2c | 280 | 120 | 80 |
| 2d | 160 | 240 | 63 |

## Patentansprüche

1. Verfahren zur Herstellung von Tetrachlor-3-imino-isoindolin-1-on (I)

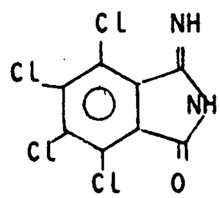

(I)

oder dessen Lithium-, Natrium- oder Kaliumsalz (Ia) durch Umsetzung von Tetrachlor-orthophthalodinitril (II)

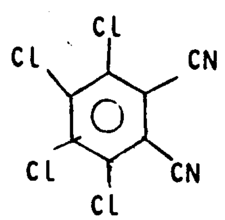

(II)

mit Lithium-, Natrium- oder Kaliumhydroxid in einem Alkohol als Lösungsmittel und gewünschtenfalls Freisetzung von (I) aus (Ia) mittels verdünnter Säure, dadurch gekennzeichnet, daß man hierbei tert.-Butanol mit einem Wassergehalt von O bis 5 Gew.-% als Lösungsmittel verwendet.

2. Verfahren gemäß Anspruch 1, dadurch gekennzeichnet, daß man feinteiliges Alkalimetallhydroxid in einer Menge von 1,0 bis 5,0 mol pro Mol Tetrachlor-ortho-phthalodinitril verwendet.

3. Verfahren gemäß Anspruch 1, dadurch gekennzeichnet, daß man die 5-bis 15-fache Gewichtsmenge tertiär-Butanol bezogen auf Tetrachlor-ortho-phthalodinitril verwendet.

4. Verfahren gemäß Anspruch 1, dadurch gekennzeichnet, daß das tertiär-Butanol einen Wassergehalt von weniger als 5 Gew. % aufweist.

5. Verfahren gemäß Anspruch 1, dadurch gekennzeichnet, daß man die Umsetzung bei einer Temperatur von 26 - 80 °C durchführt.

6. Verfahren gemäß Anspruch 1, dadurch gekennzeichnet, daß man das nach der Umsetzung und Abtrennung des Reaktionsproduktes anfallende tertiär-Butanol ohne Reinigung für weitere Umsetzungen wiederverwendet.

## Claims

1. A process for the preparation of tetrachloro-3-iminoisoindolin-1-one (I)

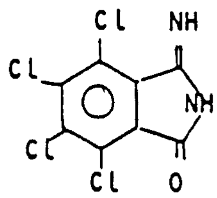

(I)

or its lithium, sodium or potassium salt (Ia) by reacting tetrachloro-ortho-phthalodinitrile (II)

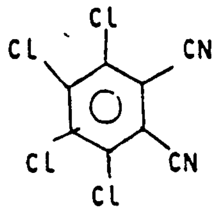

(II)

with lithium hydroxide, sodium hydroxide or potassium hydroxide in an alcohol as the solvent and, if desired, liberating (I) from (Ia) by means of a dilute acid, wherein the solvent used here is tert-butanol with a water content of from 0 to 5% by weight.

**2.** A process as claimed in claim 1, wherein finely divided alkali metal hydroxide is used in an amount of from 1.0 to 5.0 moles per mole of tetrachloro-ortho-phthalodinitrile.

**3.** A process as claimed in claim 1, wherein the weight of tert-butanol used is from 5 to 15 times the weight of tetrachloro-ortho-phthalodinitrile.

**4.** A process as claimed in claim 1, wherein the tert-butanol has a water content of less than 5% by weight.

**5.** A process as claimed in claim 1, wherein the reaction is carried out at 26-80 ° C.

**6.** A process as claimed in claim 1, wherein the tert-butanol obtained after the reaction and isolation of the reaction product is re-used for further reactions without purification.

**Revendications**

**1.** Procédé de préparation de tétrachloro-3-imino-isoindoline-1-one (I)

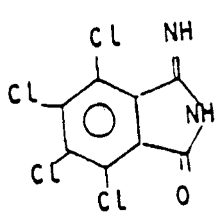

(I)

ou de sels de lithium, de sodium ou de potassium (Ia) de celle-ci, par réaction de tétrachloro-orthophtalodinitrile (II)

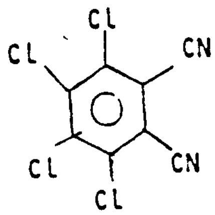

(II)

avec de l'hydroxyde de lithium, de sodium ou de potassium dans un alcool servant de solvant et, au besoin, par libération de (I) à partir de (Ia) au moyen d'un acide dilué, caractérisé en ce qu'on utilise, comme solvant, du tert.-butanol ayant une teneur en eau de 0 à 5% en poids.

**2.** Procédé selon la revendication 1, caractérisé en ce qu on utilise un hydroxyde de métal alcalin finement divisé, dans une quantité de 1,0 à 5,0 moles par mole de tétrachloro-orthophtalodinitrile.

**3.** Procédé selon la revendication 1, caractérisé en ce qu'on utilise une quantité en poids de tert.-butanol de 5 à 15 fois celle du tétrachloro-orthophtalodinitrile.

**4.** Procédé selon la revendication 1, caractérisé en ce que le tert.-butanol présente une teneur en eau de moins de 5% en poids.

**5.** Procédé selon la revendication 1, caractérisé en ce qu'on conduit la réaction à une température de 26 à 80 ° C.

**6.** Procédé selon la revendication 1, caractérisé en ce qu'on réutilise sans purification, pour d'autres réactions, le tert.-butanol obtenu après la réaction et la séparation du produit réactionnel.